(19) Europäisches Patentamt
European Patent Office
Office européen des brevets

(11) Publication number: **0 082 344**
**B1**

(12) **EUROPEAN PATENT SPECIFICATION**

(45) Date of publication of patent specification: **18.09.85**

(51) Int. Cl.[4]: **C 07 C 51/16,** C 07 C 59/245

(21) Application number: **82110913.9**

(22) Date of filing: **25.11.82**

(54) **Process for preparing beta-hydroxy-beta-methylglutaric acid.**

(30) Priority: **11.12.81 US 329654**

(43) Date of publication of application:
**29.06.83 Bulletin 83/26**

(45) Publication of the grant of the patent:
**18.09.85 Bulletin 85/38**

(84) Designated Contracting States:
**BE DE FR GB IT LU NL**

(56) References cited:
**DE-A-2 853 394**
**DE-C- 709 668**
**GB-A-1 068 650**

**CHEMICAL ABSTRACTS, vol. 95, no. 17, 26th October 1981, page 314, no. 146792t, Columbus Ohio (USA); H. OHTA et al.: "Rnantiotopically selective oxidation of 1,5-diols with gluconobacters. Preparation of (S)-mevalonolactone"**

(73) Proprietor: **AMERICAN HOECHST CORPORATION**
**Route 202-206 North**
**Somerville, N.J. 08876 (US)**

(72) Inventor: **Metzner, Ernest Kurt**
**13745 Durango Drive**
**Del Mar California 92014 (US)**

(74) Representative: **Vossius Vossius Tauchner Heunemann Rauh**
**Siebertstrasse 4 P.O. Box 86 07 67**
**D-8000 München 86 (DE)**

**EP 0 082 344 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

β-Hydroxy-β-methylglutaric acid (HMG) is a known compound with hypolipidemic and hypocholesterolemic activity; see Yusufi et al., Atherosclerosis, *20*, 517 (1974) and U.S. Patent 3,629,449.

The synthesis of this biologically important compound has been primarily accomplished by (1) oxidation of diallylmethyl carbinol or 3-hydroxy-3-methyl-hex-5-enoic acid with ozone and hydrogen peroxide (Klosterman et al, J. Am. Chem. Soc. *76*, 1229 (1954); Biochemical Preparations, *6*, 25 (1958); Tschesche et al., Liebigs Ann. der Chemie, *631*, 61 (1960) and (2) a Reformatsky reaction between ethyl acetoacetate and ethyl bromoacetate (Adams et al., J. Am. Chem. Soc. *75*, 2377 (1953).

Both of the above described synthetic methods for the preparation of HMG embrace difficulty multi-step procedures, require complicated purification schemes and produce low yields.

The present invention overcomes the disadvantages of the prior art by providing a simple one step synthesis of HMG from a readily available starting material. Use of the procedure described herein permits large scale synthesis of the HMG at very low cost.

The present invention relates to an improved process for the production of β-hydroxy-β-methylglutaric acid (HMG) which comprises oxidizing 3-methyl-1,3,5-pentanetriol with a permanganate salt in a neutral or alkaline aqueous medium. This reaction proceeds schematically as follows:

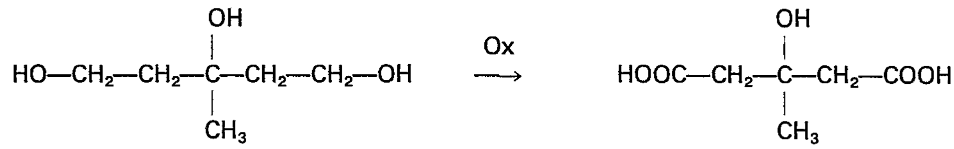


The preferred permanganate salts are sodium permanganate and potassium permanganate.

The reaction is carried out in a neutral or alkaline aqueous medium. The use of an acidic medium is a disadvantage because the yield of HMG is lower and the product is somewhat more difficult to isolate. Strongly acidic reaction conditions should be avoided due to the susceptibility of HMG to dehydration.

The reaction temperature employed herein can range from about 10°C to 110°C with a temperature range of 30°C to 45°C being preferred. A reaction time of approximately three hours is satisfactory, but the reaction time can vary from 10 minutes to several days depending on the scale of the reaction. The mole ratio of permanganate to triol can range from 3:1 to 5:1 with a ratio of 4:1 being preferred.

The reaction product HMG is recovered by conventional means. For example, insoluble manganese dioxide is removed from the reaction mixture by filtration or centrifugation. The filtrate or supernatant containing the product is then acidified to a pH of 1—2 with concentrated HCl. The resulting acidified filtrate or supernatant is then concentrated to yield a residue containing HMG which is further purified by recrystallization from a suitable solvent, such as ethyl acetate or acetonitrile.

The yields of HMG produced by the process of the present invention are excellent, i.e. yields in excess of 40% are routinely obtained.

The term "aqueous media" as used herein preferably refers to water, however minor amounts of water miscible solvents that do not interfere with the oxidation reaction may be used in combination with water.

3-Methyl-1,3,5-pentanetriol utilized herein is commercially available.

The following specific description is given to illustrate the present invention.

Example

A 2 liter reaction vessel is charged with 800 ml of water, 240 g potassium permanganate (1.52 mole) and 3 ml 19N sodium hydroxide. The resulting reaction mixture is stirred for approximately 10 minutes to dissolve most of the permanganate and the temperature is adjusted to approximately 25°C. While the resulting solution is further agitated at room temperature, 50 ml of a solution of 54 g of 3-methyl-1,3,5-pentanetriol (0.4 mole) in 150 ml of water is added. After the addition of 50 ml of the triol solution the temperature will rise to about 35°C. After approximately 10 minutes, the remaining triol solution is added dropwise over a period of 15 minutes. During the addition, the temperature is maintained at 40° to 43°C by cooling the reaction vessel in an ice bath.

After all of the triol solution has been added, the exothermic reaction will continue for about one hour. During this time, and thereafter for a period of 3 hours, the temperature is maintained at 43°C. Thereafter, the reaction mixture is heated to 80°C for approximately 10 minutes and then allowed to cool to room temperature.

The reaction mixture is filtered and the filter cake is suspended in 200 ml of water. The suspension is stirred well and then filtered. The filtrates are combined and acidified to pH 1.5 with concentrated hydrochloric acid. The acidified filtrate is then dried under reduced pressure at 60 to 65°C to obtain a dry salt cake.

The above-obtained salt cake is extracted with approximately 200 ml of acetone with the salt being removed from by filtration. Thereafter, the cake is extracted again with two 50 ml portions of acetone.

The acetone extracts are combined and dried under reduced pressure to obtain a stiff syrup which is dissolved in approximately 100 ml of boiling acetonitrile. Four grams of anhydrous magnesium sulfate and

1 gram of activated carbon are added to the hot solution which is then filtered. The resulting product is allowed to crystallize with stirring at room temperature for 3 hours. The resulting slurry is stirred with cooling to 0°C. The crystallized product is recovered by filtration, washed with ethyl acetate cooled to −20°C and dried under reduced pressure at 45°C to yield 26—28 grams of β-hydroxy-β-methylglutaric acid, m.p. 105°C—108°C. Yield 40—42%.

**Claims**

1. A process for preparing β-hydroxy-β-methylglutaric acid, characterized in that 3-methyl-1,3,5-pentanetriol is oxidized with a permanganate salt in a neutral or alkaline aqueous medium.

2. A process as defined in Claim 1 wherein said permanganate salt is selected from the group consisting of potassium permanganate and sodium permanganate.

3. A process as defined in Claim 1 wherein said oxidizing is carried out at a temperature of from about 10°C to 110°C.

4. A process as defined in Claim 3 wherein the temperature is from about 30°C to 45°C.

5. A process as defined in Claim 1 wherein the molar ratio of salt to triol is in the range of 3:1 to 5:1.

**Patentansprüche**

1. Verfahren zur Herstellung von β-Hydroxy-β-methylglutarsäure, dadurch gekennzeichnet, daß man 3-Methyl-1,3,5-pentantriol mit einem Permanganatsalz in einem neutral oder alkalischem wäßrigem Medium oxidiert.

2. Verfahren nach Anspruch 1, wobei das Permanganatsalz ausgewählt ist aus Kaliumpermanganat und Natriumpermanganat.

3. Verfahren nach Anspruch 1, wobei die Oxidation bei einer Temperatur von etwa 10°C bis 110°C durchgeführt wird.

4. Ein Verfahren nach Anspruch 3, wobei die Temperatur bei etwa 30°C bis 45°C liegt.

5. Verfahren nach Anspruch 1, wobei das Molverhältnis des Salzes zum Triol im Bereich von 3:1 bis 5:1 liegt.

**Revendications**

1. Procédé de préparation d'acide β-hydroxy-β-méthylglutarique, caractérisé en ce que du 3-méthyl-1,3,5-pentanetriol est oxydé avec un permanganate dans un milieu aqueux neutre ou alcalin.

2. Procédé suivant la revendication 1, caractérisé en ce que le permanganate est choisi dans le groupe formé par le permanganate de potassium et le permanganate de sodium.

3. Procédé suivant la revendication 1, caractérisé en ce que l'oxydation est effectuée à une température d'environ 10°C à 110°C.

4. Procédé suivant la revendication 3, caractérisé en ce que la température est d'environ 30°C à 45°C.

5. Procédé suivant la revendication 1, caractérisé en ce que le rapport molaire du sel au triol est dans la gamme de 3:1 à 5:1.